# EUROPEAN PATENT SPECIFICATION

(11) **EP 2 911 517 B1**
(45) Date of publication and mention of the grant of the patent: **27.03.2019**
(21) Application number: 13780350.8
(22) Date of filing: 22.10.2013
(51) Int. Cl.: A01N 63/04

(54) **A NOVEL IMMUNOGENIC FUNGAL EXTRACT AND PATTERN RECOGNITION RECEPTOR IN PLANTS**
NEUES IMMUNOGENES PILZEXTRAKT UND MUSTERERKENNUNGSREZEPTOR BEI PFLANZEN
NOUVEL EXTRAIT FONGIQUE IMMUNOGÈNE ET RÉCEPTEUR À RECONNAISSANCE DE MOTIF DANS DES PLANTES

(30) Priority: 23.10.2012 GB 201219017
(43) Date of publication of application: 02.09.2015
(73) Proprietor: Eberhard Karls Universität Tübingen, 72074 Tübingen (DE)
(72) Inventor: GUST, Andrea, 72119 Ammerbuch (DE); BRUNNER, Frédéric, 85354 Freising (DE); FRAITURE, Malou, 6990 Rameldange (LU); ZHANG, Weiguo, 8003 Zürich (CH)
(74) Representative: Krauss, Jan
(86) International application number: PCT/EP2013/072083
(87) International publication number: WO 2014/064111

(56) References cited:
- EP-A1- 1 671 535
- WO-A2-2006/055851
- RU-C2- 2 173 519
- GUST A A ET AL: "Plant LysM proteins: modules mediating symbiosis and immunity", TRENDS IN PLANT SCIENCE, ELSEVIER SCIENCE, OXFORD, GB, vol. 17, no. 8, 17 August 2012 (2012-08-17), pages 495-502, XP002690277, ISSN: 1360-1385, DOI: 10.1016/J.TPLANTS.2012.04.003 [retrieved on 2012-05-10]
- DATABASE UniProt [Online] 11 September 2007 (2007-09-11), "SubName: Full=Cytochrome c;", XP002718373, retrieved from EBI accession no. UNIPROT:A7E6R4 Database accession no. A7E6R4
- MARTEL MARIE-BENEDICTE ET AL: "Purification of endo polygalacturonases from Sclerotinia sclerotiorum: Multiplicity of the complex enzyme system", CURRENT MICROBIOLOGY, SPRINGER, NEW YORK, NY, US, vol. 33, no. 4, 1 January 1996 (1996-01-01), pages 243-248, XP009175388, ISSN: 0343-8651
- W. ZHANG ET AL: "Arabidopsis RECEPTOR-LIKE PROTEIN30 and Receptor-Like Kinase SUPPRESSOR OF BIR1-1/EVERSHED Mediate Innate Immunity to Necrotrophic Fungi", THE PLANT CELL ONLINE, vol. 25, no. 10, 1 October 2013 (2013-10-01), pages 4227-4241, XP055094974, ISSN: 1040-4651, DOI: 10.1105/tpc.113.117010
- DATABASE UniProt [Online] 11 May 2016 (2016-05-11), "RecName: Full=Receptor like protein 30 {ECO:0000303|PubMed:18434605}; Short=AtRLP30 {ECO:0000303|PubMed:18434605}; Flags: Precursor;", retrieved from EBI accession no. UNIPROT:Q9MA83 Database accession no. Q9MA83
- G. Wang ET AL: "A Genome-Wide Functional Investigation into the Roles of Receptor-Like Proteins in Arabidopsis", PLANT PHYSIOLOGY, vol. 147, no. 2, 11 April 2008 (2008-04-11), pages 503-517, XP055147584, ISSN: 0032-0889, DOI: 10.1104/pp.108.119487
- Weiguo Zhang: "Identification and Characterization of the Novel Fungal MAMP SsE1 and its Receptor-Like Protein (RLP30)-based Perception System in Arabidopsis", Dissertation, 26 November 2013 (2013-11-26), XP055147668, Retrieved from the Internet: URL:https://publikationen.uni-tuebingen.de /xmlui/bitstream/handle/10900/49988/pdf/Th esis_Weiguo_Zhang.pdf?sequence=1&isAllowed =y [retrieved on 2014-10-20]

## Description

### FIELD OF THE INVENTION

The present disclosure relates to a purified fungal extract (SsEl) that elicits immune responses in plants and the invention pertains to the identification of the plant receptor AtRLP30 which mediates the recognition of SsEl. Further provided is the use of AtRLP30 in plants in order to modulate the plants immune response against fungal infections with *Sclerotinia spp..*

### DESCRIPTION

The current model of the plant immune system supports the existence of two branches. One branch relies on the perception of highly conserved pathogen-associated molecular patterns (PAMPs) by membrane localized pattern recognition receptors (PRRs) resulting in the activation of intracellular signalling pathways leading to the reinforcement of the cell wall and production of anti-microbial compounds. PAMPs are per definition indispensable molecules that are characteristic to a whole class of microbes and therefore are difficult to mutate or to delete without causing a severe fitness penalty for the microbe. PAMPs are also referred to as microbe-associated molecular patterns (MAMPs), as they are not restricted to pathogenic microbes only. A well-known example is Pep-13, a surface-exposed antigenic epitope within a calcium-dependent cell wall transglutaminase that is widely distributed in pathogenic oomycetes belonging to the order of the *Perenosporales* but also present in marine *Vibrio* bacteria. Pep-13 activates defence in parsley and potato, suggesting its function as a genus-specific recognition determinant for the activation of plant immunity both in host and non-host plants.

More recently, bacterial peptidoglycan was identified as novel PAMP/MAMP triggering immune responses in *Arabidopsis thaliana.* Peptidoglycan treatment induces ion fluxes, the increase of intracellular Ca²⁺ concentrations, the production of reactive oxygen species and the phytoalexin camalexin. Transcript profiling experiments revealed that peptidoglycan affects the expression of many genes and that the re-programming of the *Arabidopsis* transcriptome overlaps well with the changes induced by the flagellin-derived flg22 peptide, a genuine PAMP/MAMP. A reverse genetic approach identified the lysin motif containing receptor-like proteins (RLP) LYM1 and LYM3 as peptidoglycan receptors that additionally require the action of a third LysM protein, the LysM-RLK CERK1.

Pattern recognition receptors (PRRs) on the plant cell surface recognize MAMPs and transduce the signal into the cell. Until now several receptors have been identified belonging to the leucine-rich repeat receptor-like kinase (LRR-RLK) and protein (LRR-RLP) family and to the LysM-RLKs/RLPs. The best studied PAMP/MAMP receptor in plants is FLS2, a receptor-like kinase protein from *Arabidopsis* with an extracellular leucine-rich-repeat domain (LRR-RLK) that detects and binds flg22, a 22 amino acid fragment from bacterial flagellin. Orthologues of FLS2 are present in tomato, tobacco, barley and rice, suggesting that perception of flagellin is an evolutionarily ancient mechanism of pathogen detection. Importantly, *Arabidopsis* T-DNA lines with a non-functional FLS2 are more susceptible to infections caused by pathogenic bacteria such as *Pto* DC3000. Recent studies have shown that FLS2 forms a complex with BAK1, another member of the LRR-RLK family in a flg22-dependent manner. Interestingly, BAK1 was initially identified as an interactor of BRI1, an LRR-RLK that recognizes the plant hormone brassinolide involved in the control of growth and development. BAK1 action is required for the response to several PAMPs/MAMPs, including flg22 and bacterial elongation factor EF-Tu in *Arabidopsis,* or bacterial Cold-shock protein and the oomycete elicitin lNFl in tobacco. The molecular mechanism underlying receptor activation involves ligand-induced conformational changes within the receptor complex followed by auto- and trans-phosphorylation events in the kinase domain of the interacting partners triggering downstream signalling. Although different PAMPs/MAMPs are perceived by different receptors, they induce common early-signalling events, including increased cytoplasmic calcium levels, MAP kinase activation, production of reactive oxygen species and induction of salicylic acid, jasmonate and ethylene biosynthesis in *Arabidopsis* plants.

Importantly, biological priming using PAMPs/MAMPs provides means for triggering plant defences in a nontransgenic manner and is already marketed for plant health strengthening in agriculture.

Wang et al. (Plant Physiology, June 2008, vol. 147 pp 503-517) describe a screen for the function of receptor like proteins in plant immunity using a library of Arabidopsis T-DNA insertion mutants.

In view of the above, it is an object of the present invention to provide novel PAMPs/MAMPs in plants and their respective receptors, which allow enhancing the resistance of plants, specifically to infections with fungal pathogens of the Sclerotiniaceae family.

The above problem is solved by the invention defined by the appended set of claims.

Herein disclosed is a plant-immunogenic fungal extract, obtainable by a process comprising the steps of,
(a) providing a culture filtrate from *Sclerotinia sclerotiorum* cells,
(b) adding said filtrate of (a) to a first cation-exchange column equilibrated with a low salt buffer A,
(c) eluting the extract with a high salt buffer B,
(d) diluting the eluted fraction of (c) with buffer A to allow for a binding to a second cation-exchange column,
(e) adding the diluted fraction of (d) to a second cation-exchange column, and eluting the plant-immunogenic fungal extract using a buffer with a salt conductivity of between, 5 - 20 mS/cm, preferably of between 8 - 16 mS/cm.

Also disclosed is a method for obtaining an immunogenic fungal extract comprising the steps of, (a) providing a culture filtrate from *Sclerotinia sclerotiorum* cells, (b) adding said filtrate of (a) to a first cation-exchange column equilibrated with a low salt buffer A, (c) eluting the extract with a high salt buffer B, (d) diluting the eluted fraction of (c) with buffer A to allow for a binding to a second cation-exchange column, (e) adding the diluted fraction of (d) to a second cation-exchange column, and eluting the plant-immunogenic fungal extract using a buffer with a salt conductivity of between, 5 - 20 mS/cm, preferably of between 8 - 16 mS/cm.

The culture filtrate provided in the step (a) of the above method may be prepared by filtrating the culture medium of a *Sclerotinia sclerotiorum* culture (about 2 to 3 weeks old) through a nylon mesh, and subsequently freeze drying the medium for 3 to 4 days. The freeze-dried material is then resuspended in buffer A (about 6 ml/g dry weight) and centrifuged to dispose of insoluble particles. The supernatant is then used as culture filtrate in the method of the present invention. Also other methods of providing a fungal culture filtrate known to the person of skill in the art are encompassed by the present invention.

For the plant immunogenic fungal extract and the method for producing the same according to the disclosure it is preferred that buffer A is 100 mM Mes buffer at pH 5.4, and/or that buffer B is 100 mM Mes buffer at pH 5.4, 0.5 M KCl. Mes is the common name for the compound 2-(N-morpholino)ethanesulfonic acid. Mes buffer is prepared according to standard procedures known to the person of skill in the art.

Further preferred is the above plant immunogenic fungal extract and the method for producing the same, wherein said first cation-exchange column has a GE Healthcare Sepharose SP™ FastFlow matrix. Most preferably said matrix is packed in a GE Healthcare XK16 column to a final bed volume of about 15 ml.

The culture filtrate may be added to said first cation-exchange column at a flow rate of 3 to 5 ml/min. Optionally, the column is then washed with buffer A.

Preferably, the elution in step (c) is performed with buffer B at a flow rate of 3-5 ml/min. Here it is preferred that a single fraction corresponding to the elution peak monitored with OD₂₈₀ₙₘ and OD₂₁₅ₙₘ is collected. Optionally, the eluted fraction may be tested in plants - such as *Arabidopsis thaliana* - for ethylene-inducing activity.

Alternatively, the extract of the disclosure can be tested for its activity to elicit typical PAMP induced defence responses in plants, such as the post translational MAP kinase activation and the transcriptional activation of immune marker genes, for example pathogenesis-related protein 1 (PR1) or flagellin-responsive kinase 1 (FRK1). The disclosure shall however not to be understood to be limited to the testing using the aforementioned defence reactions. Also other immune gene expressions or biochemical reactions typical for a pathogen infection can be monitored in order to test the extract of the invention for its activity.

In a further preferred embodiment, said second cation-exchange column is a GE Healthcare Source 15S 4.6/100 PE column, which is preferably equilibrated in advance with buffer A. The plant immunogenic fungal extract and the method for producing the same is preferred, wherein in step (d) the eluted fraction is diluted about 10 fold with buffer A.

In another preferred embodiment the diluted fraction obtained in step (d) is loaded to the second cation-exchange column at a flow rate of 0.5 to 1.5 ml/min. After loading the second cation-exchange column with the diluted fraction, the column is preferably washed with buffer A.

For the final elution step (e) it is preferred to use a flow rate of 0.5 to 1 ml/min of said elution buffer.

In one preferred embodiment, the elution is performed using a linear salt gradient of buffer B (for example 0% to 60% in 40 column volumes) and collecting a series of 500 µl fractions over this gradient. Subsequently those fractions that were eluted by a salt conductivity of between 5 - 20 mS/cm, preferably of between 8 - 16 mS/cm, contain the extract of the invention. Alternatively, all fractions eluted by a salt conductivity of between 5 - 20 mS/cm, preferably of between 8 - 16 mS/cm, are pooled to obtain the extract of the invention.

After step (e) the eluted fraction, which constitutes the extract of the invention, may optionally be tested for the ethylene-inducing activity, for example according to the methods described in the examples of the present invention.

In the context of the present disclosure it is preferred that chromatography methods are performed on a GE Healthcare ÄKTA Explorer FPLC system cooled at 4 °C and guided by the GE Healthcare Unicorn software.

The immunogenic fungal extract of the disclosure may comprise at least one of the proteins selected from Cytochrome C (A7E6R4), N-acetyltransferase (A7F941), Rho-GDP dissociation inhibitor (A7ET57), Polyubiquitin (A7E4E9), Protein disulfide isomerase (A7EDH2) or Pectin esterase (A7EXV0). Even more preferred is that said fungal extract comprises at least two of said above mentioned proteins, preferably at least three of said proteins, and most preferably all of said proteins. Accession numbers in brackets are UniProtKB/TrEMBL entries.

The disclosure further provides an immunogenic fungal extract which comprises at least one of the proteins selected from Cytochrome C (A7E6R4), N-acetyltransferase (A7F941), Rho-GDP dissociation inhibitor (A7ET57), Polyubiquitin (A7E4E9), Protein disulfide isomerase (A7EDH2) or Pectin esterase (A7EXV0). Even more preferred is that said fungal extract comprises at least two of said above mentioned proteins, preferably at least three of said proteins, and most preferably all of said proteins.

The immunogenic fungal extracts of the disclosure are in one aspect used in the activation, enhancement or priming of an immune response in a plant.

The disclosure further provides the use of an isolated protein selected from Cytochrome C (A7E6R4), N-acetyltransferase (A7F941), Rho-GDP dissociation inhibitor (A7ET57), Polyubiquitin (A7E4E9), Protein disulfide isomerase (A7EDH2) or Pectin esterase (A7EXV0), or an active fragment or homolog thereof, for the activation, enhancement or priming of an immune response in a plant.

A homologous protein in the context of the present invention shall denote a protein having an amino acid sequence with 50%, 55%, 60%, 65%, 70%, 75%, 80%, 85%, 90%, 95%, 96%, 97%, 98%, or preferably 99% sequence identity to one of the proteins selected from Cytochrome C (A7E6R4), N-acetyltransferase (A7F941), Rho-GDP dissociation inhibitor (A7ET57), Polyubiquitin (A7E4E9), Protein disulfide isomerase (A7EDH2) or Pectin esterase (A7EXV0). According to the present invention, active fragments of these proteins or homologs thereof are such parts of the proteins which have the activity of inducing an immune response in a plant against a fungal infection, preferably against an infection by Sclerotiniaceae, such as an infection of a plant with *Sclerotinia sclerotiorum* or *Botrytis cinerea.*

Thus, a further aspect of the disclosure also relates to the use of a nucleic acid encoding for a protein selected from Cytochrome C (A7E6R4), N-acetyltransferase (A7F941), Rho-GDP dissociation inhibitor (A7ET57), Polyubiquitin (A7E4E9), Protein disulfide isomerase (A7EDH2) or Pectin esterase (A7EXV0), or an active fragment or homolog thereof, for the activation, enhancement or priming of an immune response in a plant.

A homologous nucleic acid in the context of the present invention shall denote a nucleic acid having an nucleic acid sequence with 50%, 55%, 60%, 65%, 70%, 75%, 80%, 85%, 90%, 95%, 96%, 97%, 98%, or preferably 99% sequence identity to one of the nucleic acids encoding for a protein selected from Cytochrome C (A7E6R4), N-acetyltransferase (A7F941), Rho-GDP dissociation inhibitor (A7ET57), Polyubiquitin (A7E4E9), Protein disulfide isomerase (A7EDH2) or Pectin esterase (A7EXV0). According to the present invention, active fragments of these proteins or homologs thereof are such parts of the proteins which have the activity of inducing an immune response in a plant against a fungal infection, preferably against an infection by Sclerotiniaceae, such as an infection of a plant with *Sclerotinia sclerotiorum* or *Botrytis cinerea.*

The invention pertains to a method for modulating the resistance of a plant to a fungal infection by *Sclerotiniaceae,* comprising, modulating in said plant the expression of a protein comprising an amino acid sequence of at least 60% identity to the sequence shown in SEQ ID No. 1, wherein modulating the resistance of a plant constitutes either an increase or a decrease of the resistance of a plant, wherein an increase of expression of said protein results in the increase of the resistance of said plant to a pathogen infection, and wherein the decrease of expression results in a decrease of the resistance of said plant to a pathogen infection.

In some embodiments of the invention, the "modulating the resistance of a plant to a pathogen infection" constitutes either an increase or a decrease of the resistance of a plant. On the other hand, "modulating in said plant the expression of a protein" denotes either an increase of the expressed protein product in said plant compared to a non-treated control plant, or a decrease of expressed protein product in said plant compared to a non-treated control plant. Alternatively, not the expression of said protein is modulated but the activity of the protein. In this respect the modulation of the activity of a protein means either an increase or decrease of the biochemical/biological function of said protein. Such modulation of activity can be induced for example by introducing into a plant a mutated protein which displays altered biochemical characteristics compared to the wild-type protein.

In this aspect an increase of expression/activity of said protein of the invention results in the increase of the resistance of said plant to a pathogen infection. The decrease of expression/activity results in a decrease of the resistance of said plant to a pathogen infection.

It was furthermore surprisingly found that the signalling through AtRLP30 furthermore is supplemented by the co-receptor protein BRI1-associated kinase 1 (BAK1). Hence, one preferred method of the invention further comprises, in addition to the modulation of AtRLP30 or its homologs, modulating in said plant the expression of a protein comprising an amino acid sequence of at least 50%, 60%, 70%, 80%, 90%, 95%, 98%, 99% or 100% identity to the amino acid sequence of BAK1, wherein an increase of expression of said protein results in the increase of the resistance of said plant to a pathogen infection, and wherein the decrease of expression results in a decrease of the resistance of said plant to a pathogen infection. Thus, for the methods of the present invention, the modulation of expression or activity of both BAK1 and AtRLP30, or homologs thereof, allows for an even enhanced modulation of the immune response of a plant, specifically an immune response against a fungal infection.

In the context of the invention the preferred BAK1 is the protein BAK1 from *Arabidopsis thaliana.*

In preferred embodiments of the invention the expression of said protein as described above in said plant is increased by ectopic expression of said protein. On the other hand, the expression of said protein in said plant is decreased by, for example, directed mutagenesis, RNA interference or RNA mediated DNA methylation.

RNA mediated silencing mechanisms can interfere with gene expression at different levels: some RNA-directed mechanisms act at a post-transcriptional level through degradation of targeted messenger RNAs. However, dsRNA-derived species can also direct changes in the chromatin structure of DNA regions with which they share sequence identity. For example, plants use such RNA species to lay down cytosine methylation imprints on identical DNA sequences, providing a fundamental mark for the formation of transcriptionally silent heterochromatin. This process is generally referred to as RNA-directed DNA methylation (RdDM).

RdDM is initiated by the presence of double stranded RNA (dsRNA) molecules in the cell nucleus. They potentially trigger the *de novo* methylation of all cytosine bases that are located in DNA regions complementary to the sequence of the RNA double strand. As a consequence, in mammals and in plants, the methylated DNA positions serve as flags for the remodelling of the surrounding chromatin in a way, that dense heterochromatin can be formed at these loci. Due to the dense chromatin environment other proteins are prohibited from contacting the DNA. In particular transcription factors or components of the transcriptional machinery cannot assemble on the methylated promoter sequences and thus no transcription can occur in these regions. In effect, genes that have methylated regulatory sequences are less transcribed and therefore less expressed.

Preferably, the modulation of the expression of AtRLP30 and/or BAK1, or their homologs of the invention is done by RNA mediated DNA methylation targeting a sequence selected from, but is not limited to, an endogenous regulatory sequence that regulates plant DNA transcription. As used herein, the term "regulatory sequence" means a nucleotide sequence that, when operatively linked to a coding region of a gene, affects transcription of the coding region such, that a ribonucleic acid (RNA) molecule is transcribed from the coding region. A regulatory element generally can increase or decrease the amount of transcription of a nucleotide sequence, for example, a coding sequence, operatively linked to the element with respect to the level at which the nucleotide sequence would be transcribed absent the regulatory element. Regulatory elements are well known in the art and preferably include promoters, enhancers, silencers, inactivated silencer intron sequences, 3'-untranslated or 5'-untranslated sequences of transcribed sequence, preferably a poly-A signal sequence, or other protein or RNA stabilizing elements, insulators which restrict the regulatory effect of these sequences to defined regions, or other gene expression control elements known to regulate gene expression or the amount of expression of a gene product. A regulatory element can be isolated from a naturally occurring genomic DNA sequence or can be synthetic, for example, a synthetic promoter.

The terms "polynucleotide", "oligonucleotide," and "nucleic acid sequence" are used interchangeably in the context of the present invention to refer to a polymeric (two or more monomers) form of nucleotides of any length, either ribonucleotides or deoxyribonucleotides. Although nucleotides are usually joined by phosphodiester linkages, the term also includes polymers containing neutral amide backbone linkages composed of aminoethyl glycine units. The terms are used only to refer to the primary structure of the molecule. Thus, the term includes double stranded and single stranded DNA molecules as above. It will be recognized that such polynucleotides can be modified, for example, by including a label such as a radioactive, fluorescent or other tag, by methylation, by the inclusion of a cap structure, by containing a substitution of one or more of the naturally occurring nucleotides with a nucleotide analogue, by containing an internucleotide modification such as having uncharged linkages (e.g., methyl phosphonates, phosphotriesters, phosphoramidates, carbamates, or the like), by containing a pendant moiety such as a protein (e.g., a nuclease, toxin, antibody, signal peptide, poly-L-lysine, or the like), by containing an intercalator such as acridine or psoralen, by containing a chelator, which can be a metal such as boron, an oxidative metal, or a radioactive metal, by containing an alkylator, or by having a modified linkage (e.g., an alpha anomeric nucleic acid).

Preferred polynucleotide species according to the present invention are selected from ssDNA, dsDNA tDNA, ssRNA, dsRNA, shRNA, siRNA, and mRNA. Preferably, the polynucleotide is a DNA that codes for a dsRNA molecule, preferably a dsRNA-hairpin. DsRNA hairpins are preferably generated by expression of a DNA construct that encodes contiguous sense and anti-sense sequences that are separated by a spacer. Upon transcription of such a construct, the generated ssRNA molecule forms a double strand by base pairing of the sense and antisense sequences.

For the invention as described herein, a *Sclerotiniaceae* infection is preferably an infection of a plant with *Sclerotinia sclerotiorum.*

Plants which are preferably used in the context of the present invention, or which are preferred targets for the immunogenic extracts are corn (*Zea mays*), Brassica sp. (e.g., *B. napus, B. rapa, B. juncea*), alfalfa (*Medicago sativa*), rye (*Secale cereale*), sorghum (*Sorghum bicolor, Sorghum vulgare*), sunflower (*Helianthus annuus*), safflower (*Carthamus tinctorius*), wheat (*Triticum aestivum*), soybean (*Glycine max*), tobacco (*Nicotiana tabacum*), potato (*Solanum tuberosum*), peanuts (*Rachis hypogaea*), cotton (*Gossypium barbadense, Gossypium hirsutum*), sweet potato (*Ipomoea batatus*), cassava (*Manihot esculenta),* coffee (*Cofea spp.),* coconut (*Cocos nucifera*), pineapple (*Ananas comosus*), citrus trees (*Citrus spp*.), cocoa (*The-obroma cacao*), tea (*Camellia sinensis*), banana (*Musa spp.*), avocado (*Perseaultilane*), fig (*Ficuscasica*), guava (*Psidium guava),* mango (*Mangifera indica*), olive (*Olea europaea*), papaya (*Carica papaya*), cashew(Anacardium occidentale), macadamia (*Macadamia integri-folia*),ahnond (*Prunus amygdalus*)*,* sugar beets (*Beta vulgaris*), sugarcane (*Saccharum spp*.), oats, duckweed (*Lemna*), barley, tomatoes (*Lycopersicon esculentum*), lettuce (e. g., *Lactuca sativa*), green beans (*Phaseolus vulgaris*), lima beans (*Phaseoluslimensis*), peas (*Lathyrus spp*.), and members of the genus Cucumis such as cucumber (*C sativus*), cantaloupe (*C*. *can-talupensis*), and musk melon (*C. melo*). Ornamentals such as azalea (*Rhododendron spp*.), hydrangea (*Macrophylla hydrangea*), hibiscus (*Hibiscus rosasanensis*), roses (*Rosa spp.*), tulips (*Tulipa spp.*), daffodils (*Narcissus spp*.), petunias (*Petunia hybrida*), carnation (*Dian-thus caryophyllus*), poinsettia (*Euphorbia pulcherrima*), and chrysanthemum are also included. Additional ornamentals within the scope of the invention include impatiens, Begonia, Pelargonium, Viola, Cyclamen, Verbena, Vinca, Tagetes, Primula, Saint Paulia, Agertum, Amaranthus, Antihirrhinum, Aquilegia, Cineraria, Clover, Cosmo, Cowpea, Dahlia, Datura, Delphinium, Gerbera, Gladiolus, Gloxinia, Hippeastrum, Mesembryanthemum, Salpiglossos, and Zinnia. Conifers that may be employed in practicing the present invention include, for example, pines such as loblolly pine (*Pinus taeda*), slash pine (*Pinus elliotii*), ponderosa pine (*Pinus ponderosa*), lodgepole pine (*Pinus contorta*), and Monterey pine (*Pinus radiata*), Doug-las-fir (*Pseudotsuga menziesii*); Western hemlock (*Tsugaultilane*); Sitka spruce (*Picea glau-ca*); redwood (*Sequoia sempervirens*); true firs such as silver fir (*Abies amabilis*) and balsam fir (*Abies balsamea*); and cedars such as Western red cedar (*Thuja plicata*), and Alaska yellow-cedar (*Chamaecyparis nootkatensis*), preferably wherein the plant is susceptible to an infection with *Sclerotinia spp* or *Botrytis spp.*

The problem of the present invention is further solved by a method for producing a transgenic plant having enhanced resistance to a fungal infection by *Sclerotiniaceae,* comprising the steps of (i) transforming a plant or plant cell with a nucleotide sequence encoding for a AtRLP30 or AtRLP30-like protein comprising an amino acid sequence of at least 60%, identity to SEQ ID No 1. In preferred embodiments of the invention, the nucleotide sequence encoding for a AtRLP30 or AtRLP30-like protein comprising an amino acid sequence of at least 60%, 70%, 80%, 90%, 95%, 98%, 99% or 100% identity to SEQ ID No 1 is comprised in an expression vector that allows for the expression of the polynucleotide in a plant.

In preferred embodiments of the invention, the any methods for the production of plants as described herein are not essentially biological processes. More preferably methods for the production of plants as described herein do not contain or consist of any steps of sexually crossing the whole genomes of plants and of subsequently selecting plants.

In still another aspect the disclosure pertains to a gene, comprising a nucleotide sequence encoding for a AtRLP30 or AtRLP30-like protein (homolog) comprising an amino acid sequence of at least 50%, 60%, 70%, 80%, 90%, 95%, 98%, 99% or 100% identity to SEQ ID No 1.

The term "gene" as used in the context of the invention describes any DNA sequence element that can be transcribed into RNA and that might encode for a heritable trait in an organism. Most genes are protein coding genes, wherein the nucleotide sequence of the gene codes for the amino acid sequence of the protein product. However, other genes might code for RNAs which are not translated into proteins - so called non-coding RNAs (ncRNAs). For example, ncRNA genes encode for transfer RNAs (tRNAs), or structural RNAs as found in large protein complexes like the ribosome (rRNAs). Further, the term "gene" includes coding regions for small none-coding RNAs species. Small non-coding RNA genes include snoRNAs, mi-croRNAs, siRNAs and piRNAs and long ncRNAs that include examples such as Xist and HOTAIR.

Yet another aspect of the disclosure relates to an expression cassette characterized in that the expression cassette allows for the expression of a gene according to the invention.

A further preferred aspect of the present disclosure is then directed at a vector comprising a nucleic acid according to the present invention, for example a gene or an expression cassette according to the present invention. A vector within the meaning of the present invention is a protein or a nucleic acid or a mixture thereof which is capable of being introduced or of introducing the polynucleotides comprised into a cell. It is preferred that the proteins encoded by the introduced nucleic acid are expressed within the cell upon introduction of the vector.

In a preferred embodiment, the vector of the present disclosure comprises recombinant vectors, plasmids, phagemids, phages, cosmids, viruses, in particular but not limited to virus-derived amplicon vectors, potato virus X based vectors, tobacco rattle virus based vectors, geminivirus-based vectors such as cabbage leaf curl virus and barley stripe mosaic virus based vectors and vectors based on satellite viruses (reviewed in Curtin, S.J., Wang, M.-B., Watson, J.M., Roffey, P., Blanchard, C.L. and Waterhouse, P.M.. (2007), chapter 12, p 291-332 in "Rice Functional Genomics; Challenges, Progress and Prospects". Upadhyaya, Narayana M. (Ed.), ISBN: 978-0-387-48903-2), virosomes, and nucleic acid coated particles, in particular gold spheres.

The term "recombinant nucleic acid molecule" refers to a polynucleotide produced by human intervention. A recombinant nucleic acid molecule can contain two or more nucleotide sequences that are linked in a manner such that the product is not found in a cell in nature. In particular, the two or more nucleotide sequences can be operatively linked and, for example, can encode a fusion polypeptide, or can comprise a nucleotide sequence and a regulatory element. A recombinant nucleic acid molecule also can be based on, but different, from a naturally occurring polynucleotide, for example, a polynucleotide having one or more nucleotide changes such that a first codon, which normally is found in the polynucleotide, is replaced with a degenerate codon that encodes the same or a conservative amino acid, or such that a sequence of interest is introduced into the polynucleotide, for example, a restriction endonuclease recognition site or a splice site, a promoter, a DNA replication initiation site, or the like.

Preferred is a recombinant vector according to the present invention, which is an expression vector, optionally comprising one or more genes to be expressed. Preferably, said expression is driven by a regulatory sequence (or sequences). A regulatory sequence can be isolated from a naturally occurring genomic DNA sequence or can be synthetic, for example, a synthetic promoter.

Such expression vectors of the present disclosure are preferably used in those embodiments in which the expression of a protein of the invention is increased in order to modulate the resistance of said transformed plant. Preferably the resistance against a pathogenic infection, most preferably the infection with a fungal pathogen as described herein before.

Regulatory sequences can be constitutively expressed regulatory sequences, which maintain gene expression at a relative level of activity (basal level), or can be regulated regulatory sequences. Constitutively expressed regulatory sequence can be expressed in any cell type, or can be tissue specific, which are expressed only in particular cell types, phase specific, which are expressed only during particular developmental or growth stages of a plant cell, or the like. A regulatory sequence such as a tissue specific or phase specific regulatory sequences or an inducible regulatory sequence useful in constructing a recombinant polynucleotide or in a practicing a method of the invention can be a regulatory sequence that generally, in nature, is found in a plant genome. However, the regulatory sequence also can be from an organism other than a plant, including, for example, from a plant virus, an animal virus, or a cell from an animal or other multicellular organism.

A preferred regulatory sequence useful for expression of polynucleotides of the disclosure is a promoter element. Useful promoters include, but are not limited to, constitutive, inducible, temporally regulated, developmentally regulated, spatially-regulated, chemically regulated, stress-responsive, tissue-specific, viral and synthetic promoters. Promoter sequences are known to be strong or weak. A strong promoter provides for a high level of gene expression, whereas a weak promoter provides for a very low level of gene expression. An inducible promoter is a promoter that provides for the turning on and off of gene expression in response to an exogenously added agent, or to an environmental or developmental stimulus. A bacterial promoter can be induced to varying levels of gene expression depending on the level of isothiopropyl galactoside added to the transformed bacterial cells. An isolated promoter sequence that is a strong promoter for heterologous nucleic acid is advantageous because it provides for a sufficient level of gene expression to allow for easy detection and selection of transformed cells and provides for a high level of gene expression when desired.

The choice of promoter will vary depending on the temporal and spatial requirements for expression, and also depending on the target species. In some cases, expression in multiple tissues is desirable. While in others, tissue-specific, e.g., leaf-specific, seed-specific, petal-specific, anther-specific, or pith-specific, expression is desirable. Although many promoters from dicotyledons have been shown to be operational in monocotyledons and vice versa, ideally dicotyledonous promoters are selected for expression in dicotyledons, and monocotyledonous promoters for expression in monocotyledons. There is, however, no restriction to the origin or source of a selected promoter. It is sufficient that the promoters are operational in driving the expression of a desired nucleotide sequence in the particular cell.

Other sequences that have been found to enhance gene expression in transgenic plants include intron sequences (e. g., from Adh 1, bronze 1, actin 1, actin 2 (WO 00/760067), or the sucrose synthase intron), poly adenylation signals in the 3' prime UTR and viral leader sequences (e.g., from TMV, MCMV and AMV). For example, a number of non-translated leader sequences derived from viruses are known to enhance expression. Specifically, leader sequences from tobacco mosaic virus (TMV), maize chlorotic mottle virus (MCMV), and alfalfa mosaic virus (AMV) have been shown to be effective in enhancing expression (e.g., Gallie et al., 1987; Skuzeski et al., 1990). Other leaders known in the art include but are not limited topicornavirus leaders, for example, EMCV leader (encephalomyocarditis virus 5'-non-coding region; Elroy-Stein et al., 1989); potyvirus leaders, for example, TEV leader (tobacco etch virus); MDMV leader (maize dwarf mosaic virus); human immunoglobulin heavy chain binding protein (BiP) leader, (Macejak et al., 1991); untranslated leader from the coat protein mRNA of AMV (AMV RNA 4; Jobling et al., 1987), TMV (Gallie et al.,1989), and MCMV (Lommel et al., 1991; see also, della Cioppa et al., 1987).

For the expression of any constructs as described herein in a plant or plant cell, the invention preferably embodies that the described polynucleotides are operable linked to a promoter and to a polyadenylation site, wherein said promoter is characterized in that it is functional in said cell of said plant. As a promoter in this context, any sequence element is sufficient that induces transcription of the downstream sequence. The minimal requirements of promoters are very well known in the art and many of such promoters are conventionally used for gene expression in plants.

In a preferred embodiment of the invention, the transformation of a plant or plant cell with any polynucleotide as described herein, is performed by a method selected from standard procedures known in the art. Transformation of plant tissue can be achieved preferably by particle bombardment (Klein et al., "High- Velocity Microprojectiles for Delivering Nucleic Acids Into Living Cells," Nature 327:70-73 (1987)), also known as ballistic transformation of the host cell, as disclosed in U.S. Patent Nos. 4,945,050, 5,036,006, and 5,100,792, all to Sanford et al., and in Emerschad et al., "Somatic Embryogenesis and Plant Development from Immature Zygotic Embryos of Seedless Grapes (Vitis vinif era) " Plant Cell Reports 14:6-12 (1995). In particle bombardment, tungsten or gold microparticles (1 to 2 Î¼m in diameter) are coated with the DNA of interest and then bombarded at the tissue using high-pressure gas. In this way, it is possible to deliver foreign nucleotides into the nucleus. Biologically active particles (e.g., dried bacterial cells containing the vector and heterologous DNA) can also be propelled into plant cells. Other variations of particle bombardment, now known or hereafter developed, can also be used. Another preferred method of stably introducing the nucleic acid construct into plant cells is to infect a plant cell with *Agrobacterium tumefaciens* or *Agrobacteriurn rhizogenes* previously transformed with the polynucleotide construct. As described above, the Ti (or RI) plasmid of *Agrobacterium* enables the highly successful transfer of a foreign nucleic acid molecule into plant cells. A preferred variation of *Agrobacterium* transformation uses vacuum infiltration in which whole plants are used (Senior, "Uses of Plant Gene Silencing," Biotechnology and Genetic Engineering Reviews 15:79-119 (1998)). Yet another referred method of introduction is fusion of protoplasts with other entities, either mini-cells, cells, lysosomes, or other fusible lipid-surfaced bodies (Fraley et al., Proc. Natl. Acad. Sci. USA 79:1859-63 (1982),). Also preferred in a method, wherein the nucleic acid molecule is introduced into the plant cells by electroporation (Fromm et al., Proc. Natl. Acad. Sci. USA 82:5824 (1985)). In this technique, plant protoplasts are electroporated in the presence of plasmids containing the expression cassette. Electrical impulses of high field strength reversibly permeabilize biomembranes allowing the introduction of the plasmids. Electroporated plant protoplasts reform the cell wall, divide, and regenerate. Other preferred methods of transformation include chemical-mediated plant transformation, microinjection, physical abrasives, viral transduction and laser beams (Senior, "Uses of Plant Gene Silencing," Biotechnology and Genetic Engineering Reviews 15 :79- 119 (1998)). The precise method of transformation is not critical to the practice of the present invention. Any method that results in efficient transformation of the host cell of choice is appropriate for practicing the present

Another aspect of the invention relates to a screening method for microbe-associated molecular patterns (PAMPs/MAMPs), comprising the method steps of (i) expressing in a plant or plant cell a protein comprising an amino acid sequence of at least 60%, identity to SEQ ID No. 1 (AtRLP30), (ii) contacting said plant or plant cell with a candidate compound, (iii) measuring the immune response of said plant or plant cell in comparison with a control plant or plant cell, wherein an elevated immune response of said plant or plant cell indicates that said candidate compound is a MAMP and wherein said MAMP is a fungal molecular pattern present in *Sclerotinia spp.* or *Botrytis ssp.*

Preferably in step (iii) of the above method of the invention the immune response of said plant or plant cell is measured by means of assessing ethylene production and/or the expression of immune responsive genes or reporter genes. Reporter genes usable in the present context are composed of an immune responsive promoter operably linked to a reporter gene that allows for an easy readout of the reporter gene expression, e.g. luciferase enzymes or fluorescent proteins. The person of skill in the art has access to a wide selection of enzymes that can be used as reporter genes.

Yet a further aspect of the invention relates to a method for purifying a MAMP present in *Sclerotinia spp.* or *Botrytis ssp,* comprising the use of a AtRLP30 protein, or extracellular parts thereof, comprising a sequence of at least 60% identity to the amino acid sequence shown in SEQ ID No. 1.

For example, for purifying the MAMP, the above protein can be coupled to a solid carrier medium, preferably to a membrane or a bead, which then allows for the selective binding of the MAMP to said protein. Then, an crude or purified extract of a pathogen cell culture is brought into contact with the carrier medium coupled to the protein of the invention. Using subsequent washing and a final elution step, a MAMP which binds selectively to the protein of the invention can be recovered.

Another aspect of the invention is a method for sensitizing a plant against fungal infections, the method comprising performing in said plant a method according to the invention as described herein before.

The present invention will now be further described in the following examples with reference to the accompanying figures and sequences, nevertheless, without being limited thereto. In the Figures and Sequences,

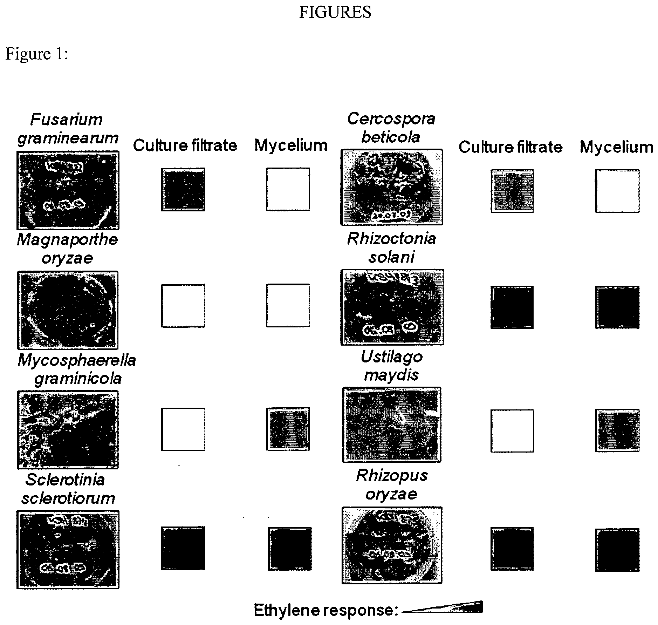

- **Figure 1:**: Ethylene response of *Arabidopsis* to extracts from major fungal crop pathogens. *Arabidopsis* leaf pieces were treated with culture filtrate or soluble mycelial extract from eight fungal species. After 3 hours, ethylene production was measured by gas chromatography. The strength of the response to the extracts is indicated by a colour code ranging from white (production similar to non treated control) over yellow and orange (low to medium production) to red (high production).

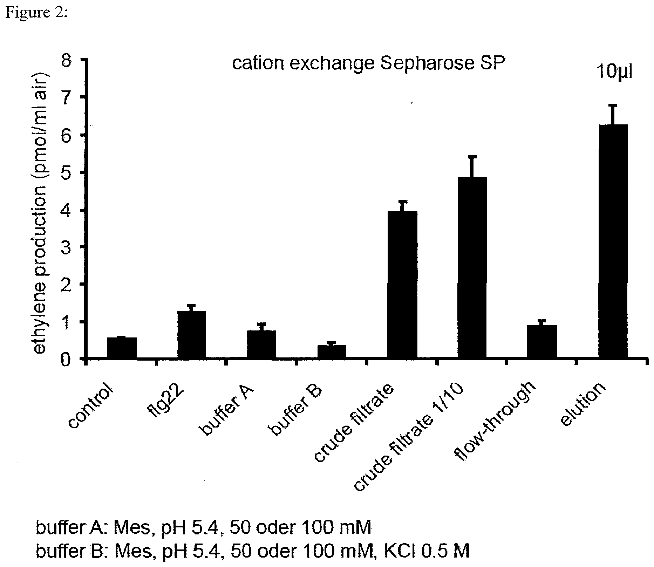

- **Figure 2:**: Ethylene response of *Arabidopsis* to SsE1-containing fraction eluted from a SP sepharose cation-exchange chomatrography column. *Arabidopsis* leaf pieces were treated with 10 µl of the SP-sepharose bound fraction that was eluted with 0.5 M KCl (Buffer B). After 3 hours, ethylene production was measured by gas chromatography. 10 µl Buffer A (50/100 mM Mes pH 5.4) and 10 µl Buffer B (50/100 mM Mes pH 5.4, 0,5 M KCl) were used as negative controls. 10 µl undiluted and 10x diluted culture filtrate (crude filtrate) were used as positive controls for ethylene production. The unbound SP-Sepharose fraction has no ethylene-inducing activity (flow-through).

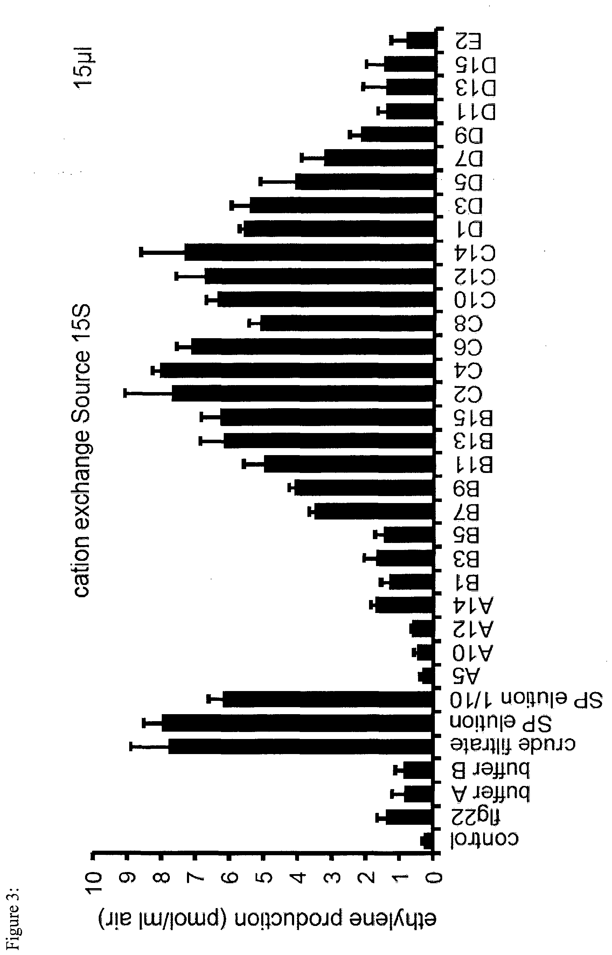

- **Figure 3:**: Ethylene response of *Arabidopsis* to SsE1-containing fractions eluted from a Source 15S cation-exchange chomatrography column. *Arabidopsis* leaf pieces were treated with 15 µl of the Source 15S bound fraction that was eluted with a gradient of 0-0.5 M KCl. 500 µl samples were collected. After 3 hours, ethylene production was measured by gas chromatography. 15 µl Buffer A (50/100 mM Mes pH 5.4) and 15 µl Buffer B (50/100 mM Mes pH 5.4, 0,5 M KCl) were used as negative controls. 15 µl undiluted culture filtrate (crude filtrate) as well as 15 µl from the active SP-sepharose fraction (SP elution), both undiluted and 10 x diluted, were used as positive controls for ethylene production. SsEl-containing fractions (B7-D7) elute at a salt conductivity value between 8 and 16 mS/cm.

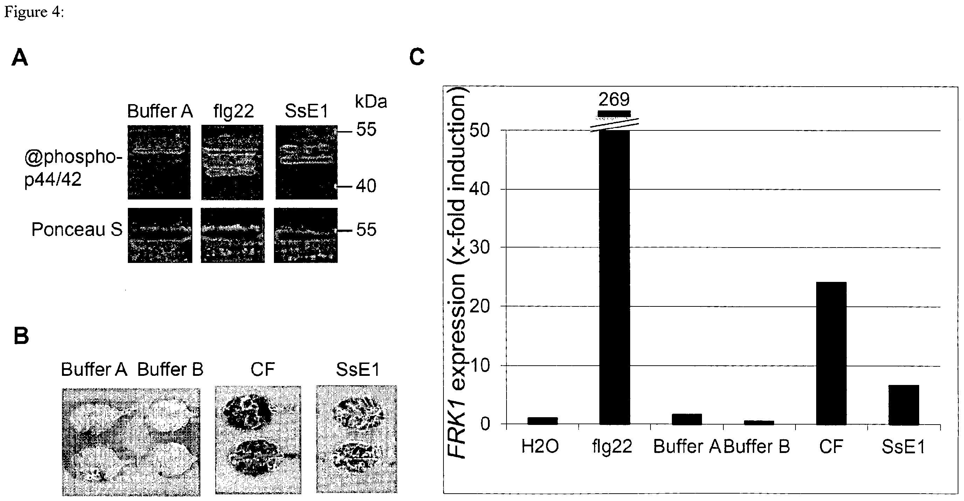

- **Figure 4:**: The *Sclerotinia sclerotiorum* elicitor fraction SsE1 generates an immune response in *Arabidopsis.* (A) Immunoblotting of activated MAPK with antiphospho p44/p42 antibody in *Arabidopsis* leaf extracts. Leaf samples were collected 10 minutes after infiltration with flg22 or SsE1. Ponceau S Red staining served as a loading control. (B) GUS activity in pPR1::GUS transgenic *Arabidopsis* plants. Leaves were infiltrated with *Sclerotinia* crude filtrate (CF); SsEl fraction or buffer and collected 24 hours later for histochemical GUS staining (C) Transcriptional profiling of *FRK1* by quantitative real-time PCR. *Arabidopsis* leaves were infiltrated with flg22, CF, SsE1, buffer or water and collected 6 hours after treatment. Expression data is normalised to the levels of *Actin* transcript and is presented as fold induction compared to the water-treated control. Buffer A = 50 mM Mes pH 5.4; Buffer B = 50 mM Mes pH 5.4, 500 mM KCI.

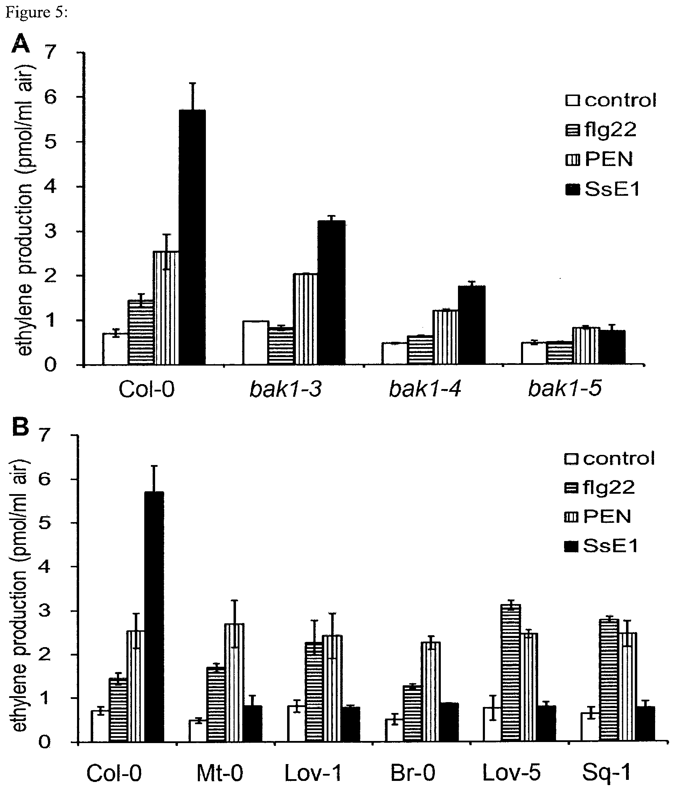

- **Figure 5:**: Ethylene response to SsE1 in *bakl* mutants and different *Arabidopsis* ecotypes. Leaf discs of 5 weeks old *Arabidopsis* plants were treated with 500 nM flg22 or partially purified extracts from *Penicillium* (PEN) and *Sclerotinia sclerotiorum* (SsE1). After 3 hours incubation, ethylene production was measured by gas chromatography. (A) Ethylene production in *Arabidopsis* Col-0 and three different bakl T-DNA insertion lines (B) Ethylene production in *Arabidopsis* Col-0 and five accessions insensitive to SsE1.

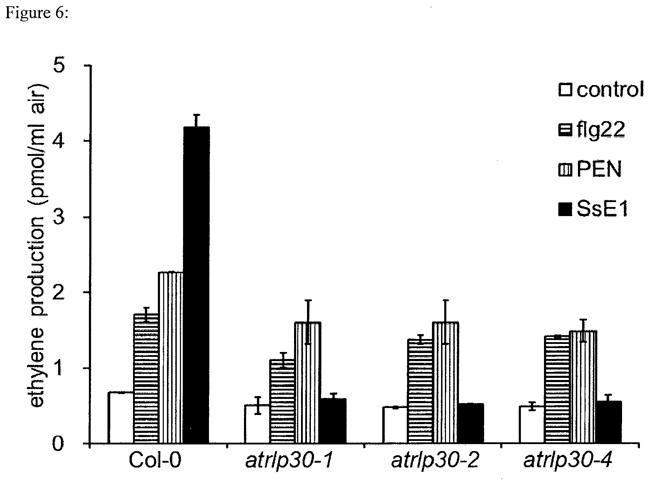

- **Figure 6:**: Ethylene response to SsE1 in *atrlp30* mutants. Three independent T-DNA insertion lines for *AtRLP30* were treated with flg22, a crude elicitor preparation from *Penicillium* (PEN) and SsE1.

SEQ ID NO. 1 shows the amino acid sequence of AtRLP30:
SEQ ID NO. 2 shows the genomic nucleic acid sequence of *AtRLP30*:

### EXAMPLES

### Example 1: Screening of Fungal Pathogens

The major fungal pathogens were screened in the context of the present invention for the presence of triggers of typical PAMP-induced defence reponses in *Arabidopsis.* The pathogens tested were *Ustilago maydis* (maize corn smut); *Magnaporthe oryzae* (rice blast); *Mycosphaerella graminicola* (STB on wheat); *Fusarium graminearum* (wheat headblight); *Cercospora beticola* (beet leafspot); *Sclerotinia sclerotiorum* (stem rot on soybean and Brassica); *Rhizopus oryzae* (post harvest decay); *Rhizoctonia solani* ("damping off in many plants). These pathogens were selected because their genomes were or are currently sequenced.

The inventors have systematically tested crude and semi-fractionated preparations of both culture filtrate and mycelium of axenically grown fungi for their ability to induce ethylene production in *Arabidopsis* leaf pieces. The strongest elicitor activities in the bio-assays were detected in the mycelium extract and culture filtrates of *Sclerotinia sclerotiorum, Rhizoctonia solani* and *Rhizopus oryzae* (Figure 1).

### Example 2: Purification of the Immunogenic Fungal Extract

Culture filtrates were first used as the elicitor source. In a two-step approach combining cation-exchange chromatography columns, the activity present in the culture filtrate of *Sclerotinia sclerotiorum* was purified to a single elicitor-containing fraction, called SsEl (*Sclerotinia sclerotiorum* Elicitor 1).

*Sclerotinia sclerotiorum* strain 1946 was purchased from Deutsche Sammlung von Mikroorganismen und Zellkulturen GmbH (DSMZ). The fungus was grown on malt-peptone agar medium (10 g malt, 2.5 g peptone, 15 g agar/L) for 3 days at room temperature in the dark. 20 to 25 1L Erlenmeyer flasks, containing 400 ml liquid malt-peptone medium (10 g malt, 2.5 g peptone) each, were then inoculated with 2-3 agar plugs. The fungus was grown for 3 weeks at room temperature in the dark without shaking. Culture medium was filtered through a nylon mesh and freeze-dried for 3 to 4 days. Freeze-dried material was re-suspended in 100 mM Mes buffer at pH 5.4 (around 6 ml/g dry weight) and centrifuged twice for 20 min at 10.000 g and 4 °C to remove insoluble particles. The resulting supernatant was used for the isolation of SsEl.

SsEl was partially purified from concentrated culture filtrate using cation exchange chromatography in a two-step approach. Chromatography was performed on a GE Healthcare ÄKTA Explorer FPLC system cooled at 4 °C and guided by the GE Healthcare Unicorn software. In a first step, a GE Healthcare XK16 column was packed with GE Healthcare Sepharose SP FastFlow matrix to a bed volume of approximately 15 ml. The column was equilibrated with buffer A (100 mM Mes buffer at pH 5.4). Concentrated culture filtrate was then loaded at a flow rate of 3 to 5 ml/min. The column was washed with buffer A and elution was performed with buffer B (100 mM Mes buffer at pH 5.4, 500 mM KCl) at a flow rate of 3-5 ml/min. A single fraction corresponding to the elution peak monitored with OD₂₈₀ₙₘ and OD₂₁₅ₙₘ was collected manually and tested for ethylene-inducing activity (Figure 2). In a second step, total eluate was diluted 10x in buffer A and loaded at a flow rate of 0.5 to 1.5 ml/min onto a GE Healthcare Source 15S 4.6/100 PE column equilibrated with buffer A. The column was washed with buffer A. SsEl was next eluted with a linear gradient of buffer B (0% to 60% in 40 column volumes) at a flow rate of 0.5 to 1 ml/min. 500 µl fractions corresponding to the whole elution peak monitored with OD₂₈₀ₙₘ and OD₂₁₅ₙₘ were collected using automated fractionation. Active fractions containing SsEl were identified by measurement of their ability to elicit ethylene production in *Arabidopsis thaliana* Col-0 leaves (Figure 3).

When infiltrated into *Arabidopsis* leaves, SsEl was able to induce typical PAMP-induced defence responses such as post-translational MAP kinase activation and the transcriptional activation of immune marker genes encoding for the pathogenesis-related protein 1 (PR1) and flagellin-responsive kinase 1 (FRK1) (Figure 4A-C).

### Example 3: Characterization of the Immunogenic Fungal Extract

In order to characterize SsEl, active fractions eluted from the Source 15S were pooled and subjected to mass spectrometry analysis using nano-LC MS/MS (B. Macek, Proteome Centre Tübingen). By this approach, several proteins were detected within the sample, which might be responsible for the immunogenic activity of SsE1. The proteins are provided in Table 1.

**Table 1:**

| **Protein ID** | **Protein name** | **Peptides** | **Mascot score** | **MW (kDa)** | **pl** |
|---|---|---|---|---|---|
| A7E6R4 | Cytochrome C | 10 | 370.22 | 12.0 | 4.3 |
| A7F941 | N-acyttransferase | 9 | 363.95 | 19.3 | 5.7 |
| A7ET57 | Rho-GDP dissociation inhibitor | 7 | 269.73 | 22.5 | 5.1 |
| A7E4E9 | Polyubiquitin | 5 | 183.40 | 34.3 | 7.4 |
| A7EDH2 | Protein disulfide isomerase | 3 | 105.35 | 39.4 | 7.2 |
| A7EXV0 | Pectin esterase | 2 | 115.36 | 34.2 | 7.3 |

### Example 4: Identification of the SsE1 Receptor

In order to identify the pattern recognition receptor mediating the immune response upon treatment with the fungal immunogenic extract of the invention, a reverse-genetic screen for receptor proteins responsive for SsEl was carried out in *Arabidopsis.* The growing body of structural and functional information on plant PRRs reveals that there are mainly two types of receptors mediating sensing of structurally different classes of microbial patterns. LRR proteins and LRR receptor kinases are thought to be implicated in host immune activation preferentially to peptide patterns whereas LysM domain proteins and LysM receptor kinases seem to mediate microbial sensing and host immunity preferentially through recognition of glycan patterns. Accordingly, the screened class of proteins was restricted to members of the LRR-RLK family which will most likely contain the SsE1 receptor.

To this end a collection of homozygous T-DNA insertion lines for 40 PAMP-induced LRR-RLKs (B. Kemmerling, ZMBP Tübingen) was screened for SsEl insensitivity. In addition the inventors obtained and tested single, *fls2 efr cerkl* triple mutants and quadruple mutants of *fls2 efr cerkl* and each one member of the LRR-RLK subfamily XII that comprises the flg22 receptor FLS2 and the EF-Tu receptor EFR (C. Zipfel, the Sainsbury laboratory Norwich, UK). None of the LRR-RLKs knock-out lines tested showed significant reduction in ethylene production when treated with SsEl except for the *bak1* mutant. Several mutant alleles of *BAK1* exist: *bak1-3* and *bak1-4* mutants are partially impaired in brassinosteroid signalling and in cell death control, whereas the newly described *bak1-5* allele is only impaired in PAMP signalling but does not affect brassinosteroid responses and cell death. While the response in *bakl-3* and *bak1-4* plants was not completely abolished, *bak1-5* plants were fully insensitive to SsEl treatment (Figure 5A).

In a parallel approach the ethylene response to SsEl treatment in 60 different *Arabidopsis* accessions was tested (Nordborg collection, Nottingham *Arabidopsis* Stock Centre). The natural genetic variation that exists between different *Arabidopsis* ecotypes may lead to the identification of ecotypes that are partially or fully insensitive to SsEl. Such ecotypes would be useful for the identification by map-based cloning of the locus responsible for SsEl perception. The best example for the successful employment of this approach was given by the identification of the flagellin-insensitive ecotype Ws-0 which is lacking a functional FLS2 receptor. Five ecotypes (Mt-0, Lov-1, Br-0, Lov-5 and Sd-1) among the 60 tested proved to be insensitive to SsEl. These ecotypes are not impaired in their ability to produce ethylene since they retained full response to flg22 or the PEN elicitor (Figure 3B). Crossings of the insensitive ecotypes Mt-0, Lov-1 and Sd-1 and analysis of the resulting F1 populations indicated that the same recessive gene(s) confer(s) responses to SsEl in all three ecotypes (data not shown).

Ethylene production was used as a trait for a genetic analysis of the sensitivity towards SsEl in Lov-1 X Col-0 crosses. Thereby, a single recessive locus that maps to the upper arm of chromosome 3 was found to be responsible for sensitivity to SsE1 (64 of 270 tested F2 plants were insensitive to the elicitor). Further fine-mapping using SSLP (Simple Sequence Length Polymorphism) and various CAPS (Cleaved Amplified Polymorphic Sequence) markers yielded a region containing four LRR-Receptor-Like Proteins (RLPs) but no members of the other LRR-containing protein families. Independent T-DNA knockout lines for each of the LRR-RLP candidates were treated with SsEl. The results showed that AtRLP30 (At3g05360) is involved in the recognition of SsEl, whereas responses to flg22 and PEN were unaltered (Figure 6). Knockout lines corresponding to the two other candidates, AtRLP32 (At3g05650) and AtRLP33 (Af3g05660), did also respond normally to SsE1

## Claims

1. Method for modulating the resistance of a plant to a fungal infection by *Sclerotiniaceae,* comprising, modulating in said plant the expression of a protein comprising an amino acid sequence of at least 60% identity to the sequence shown in SEQ ID No. 1, wherein modulating the resistance of a plant constitutes either an increase or a decrease of the resistance of a plant, wherein an increase of expression of said protein results in the increase of the resistance of said plant to a fungal infection by *Sclerotiniaceae* and wherein the decrease of expression results in a decrease of the resistance of said plant to a fungal infection by *Sclerotiniaceae.*

2. The method according to claim 1, further comprising modulating in said plant the expression of a protein comprising an amino acid sequence of at least 60% identity to BAK1, wherein an increase of expression of said protein results in the increase of the resistance of said plant to a fungal infection by *Sclerotiniaceae,* and wherein the decrease of expression results in a decrease of the resistance of said plant to a fungal infection by *Sclerotiniaceae.*

3. The method according to claim 2, wherein the expression of said protein in said plant is increased by ectopic expression of said protein.

4. The method according to claim 2, wherein the expression of said protein in said plant is decreased by mutagenesis, RNA interference or RNA mediated DNA methylation.

5. The method according to any one of claims 1 to 4, wherein said fungal infection by Sclerotiniaceae is an infection of a plant with *Sclerotinia sclerotiorum* or *Botrytis cinerea.*

6. A method for producing a transgenic plant having enhanced resistance to a fungal infection by *Sclerotiniaceae,* comprising the steps of (i) transforming a plant or plant cell with a nucleotide sequence encoding for a AtRLP30 or AtRLP30-like protein comprising an amino acid sequence of at least 60% identity to SEQ ID No 1.

7. A screening method for microbe-associated molecular patterns (PAMPs/MAMPs) present in *Sclerotinia spp* or *Botrytis ssp,* comprising the method steps of (i) expressing in a plant or plant cell a protein comprising an amino acid sequence of at least 60% identity to SEQ ID No 1 (AtRLP30), (ii) contacting said plant or plant cell with a candidate compound, (iii) measuring the immune response of said plant or plant cell in comparison with a control plant or plant cell, wherein an elevated immune response of said plant or plant cell indicates that said candidate compound is a MAMP present in *Sclerotinia spp* or *Botrytis ssp.*

8. A screening method according to claim 7, wherein in step (iii) the immune response of said plant or plant cell is measured by means of assessing ethylene production and/or the expression of immune responsive genes or reporter genes.

9. A method for purifying a MAMP present in *Sclerotinia spp* or *Botrytis ssp,* comprising the use of a AtRLP30 protein, or extracellular parts thereof, comprising a sequence of at least 60% identity to the SEQ ID No. 1.

10. A method for sensitizing a plant against fungal infections, the method comprising performing in said plant a method according to any one of claims 1 to 6.

## Patentansprüche

1. Verfahren zum Modulieren der Resistenz einer Pflanze gegen eine Pilzinfektion durch *Sclerotiniaceae,* umfassend das Modulieren der Expression eines Proteins in der Pflanze, das eine Aminosäuresequenz mit einer Identität von mindestens 60% zu der Sequenz gezeigt in SEQ ID Nr. 1 umfasst, wobei das Modulieren der Resistenz einer Pflanze entweder eine Erhöhung oder eine Verringerung der Resistenz einer Pflanze ausmacht, wobei eine Erhöhung der Expression des Proteins zu einer Erhöhung der Resistenz der Pflanze gegen eine Pilzinfektion durch *Sclerotiniaceae* führt und wobei die Verringerung der Expression zu einer Verringerung der Resistenz der Pflanze gegen eine Pilzinfektion durch *Scelrotiniaceae* führt.

2. Das Verfahren nach Anspruch 1, ferner umfassend das Modulieren der Expression eines Proteins in der Pflanze, das eine Aminosäuresequenz mit einer Identität von mindestens 60% zu BAK1 umfasst, wobei eine Erhöhung der Expression des Proteins zu einer Erhöhung der Resistenz der Pflanze gegen eine Pilzinfektion durch *Sclerotiniaceae* führt, und wobei die Verringerung der Expression zu einer Verringerung der Resistenz der Pflanze gegen eine Pilzinfektion durch *Sclerotinitaceae* führt.

3. Das Verfahren nach Anspruch 2, wobei die Expression des Proteins in der Pflanze durch ektopische Expression des Proteins erhöht wird.

4. Das Verfahren nach Anspruch 2, wobei die Expression des Proteins in der Pflanze durch Mutagenese, RNA-Interferenz oder RNA- vermittelte DNA-Methylierung verringert wird.

5. Das Verfahren nach einem der Ansprüche 1 bis 4, wobei die Pilzinfektion durch *Sclerotiniaceae* eine Infektion einer Pflanze mit *Sclerotinia sclerotiorum* oder *Botrytis cinerea* ist.

6. Ein Verfahren zur Herstellung einer transgenen Pflanze mit erhöhter Resistenz gegen eine Pilzinfektion durch *Sclerotiniaceae,* umfassend die Schritte (i) Transformieren einer Pflanze oder Pflanzenzelle mit einer Nukleotidsequenz, die für ein AtRLP30- oder AtRLP30-ähnliches Protein kodiert, das eine Aminosäuresequenz mit einer Identität von mindestens 60% zu SEQ ID Nr. 1 umfasst.

7. Ein Screening-Verfahren für mikrobenassoziierte molekulare Muster (PAMPs/MAMPs) vorhanden in *Sclerotinia spp* oder *Botrytis ssp,* umfassend die Verfahrensschritte von (i) Expression eines Proteins in einer Pflanze oder Pflanzenzelle, umfassend eine Aminosäuresequenz mit einer Identität von mindestens 60% zu SEQ ID Nr. 1 (AtRPL30), (ii) Kontaktieren der Pflanze oder Pflanzenzelle mit einer Kandidatenverbindung, (iii) Messen der Immunantwort der Pflanze oder Pflanzenzelle im Vergleich mit einer Kontrollpflanze oder Pflanzenzelle, wobei eine erhöhte Immunantwort der Pflanze oder Pflanzenzelle anzeigt, dass die Kandidatenverbindung ein MAMP ist, vorliegend in *Sclerotinia spp* oder *Botrytis ssp.*

8. Ein Screening-Verfahren nach Anspruch 7, wobei in Schritt (iii) die Immunantwort der Pflanze oder Pflanzenzelle mittels Bewertung der Ethylen-Produktion und/oder der Expression von immunspezifischen Genen oder Reportergenen gemessen wird.

9. Ein Verfahren zur Aufreinigung eines in *Sclerotinia spp* oder *Botrytis ssp* vorhandenen MAMP, umfassend die Verwendung eines AtRLP30-Proteins oder extrazelluläre Teile davon, umfassend eine Sequenz von mindestens 60% Identität zu der SEQ ID Nr. 1.

10. Ein Verfahren zum Sensibilisieren einer Pflanze gegen Pilzinfektionen, das Verfahren umfassend das Durchführen eines Verfahrens in der Pflanze nach einem der Ansprüche 1 bis 6.

## Revendications

1. Méthode de modulation de la résistance d'une plante à une infection fongique par *Sclerotiniaceae,* comprenant la modulation chez ladite plante de l'expression d'une protéine comprenant une séquence d'acides aminés présentant une identité d'au moins 60 % avec la séquence représentée dans SEQ ID No. 1, dans laquelle la modulation de la résistance d'une plante constitue soit un accroissement, soit une réduction de la résistance d'une plante, où un accroissement de l'expression de ladite protéine résulte en un accroissement de la résistance de ladite plante à une infection fongique par *Sclerotiniaceae,*
et où la réduction de son expression résulte en une réduction de la résistance de ladite plante à une infection fongique par *Sclerotiniaceae.*

2. Méthode selon la revendication 1, comprenant en outre la modulation chez ladite plante de l'expression d'une protéine comprenant une séquence d'acides aminés présentant une identité d'au moins 60 % avec BAK1, où un accroissement de l'expression de ladite protéine résulte en un accroissement de la résistance de ladite plante à une infection fongique par *Sclerotiniaceae,*
et où la réduction de son expression résulte en une réduction de la résistance de ladite plante à une infection fongique par *Sclerotiniaceae.*

3. Méthode selon la revendication 2, dans laquelle l'expression de ladite protéine chez ladite plante est accrue par l'expression ectopique de ladite protéine.

4. Méthode selon la revendication 2, dans laquelle l'expression de ladite protéine chez ladite plante est réduite par mutagenèse, interférence d'ARN ou méthylation de l'ADN médiée par l'ARN.

5. Méthode selon l'une quelconque des revendications 1 à 4, dans laquelle ladite infection fongique par *Sclerotiniaceae* est une infection de la plante par *Sclerotinia sclerotiorum* ou *Botrytis cinerea.*

6. Méthode de production d'une plante transgénique ayant une résistance accrue à une infection fongique par *Sclerotiniaceae,* comprenant les étapes de (i) transformation d'une plante ou d'une cellule végétale avec une séquence de nucléotides codant pour une protéine AtRLP30 ou assimilable à AtRLP30 comprenant une séquence d'acides aminés présentant une identité d'au moins 60 % avec SEQ ID No. 1.

7. Méthode d'identification par criblage de motifs moléculaires associés à des microbes (PAMP/MAMP) présents chez *Sclerotinia spp* ou *Botrytis ssp,* comprenant les étapes de méthode de (i) expression chez une plante ou cellule végétale d'une protéine comprenant une séquence d'acides aminés présentant une identité d'au moins 60 % avec SEQ ID No 1 (AtRLP30), (ii) mise en contact de ladite plante ou cellule végétale avec un composé candidat, (iii) mesure de la réponse immunitaire de ladite plante ou cellule végétale comparativement à une plante ou cellule végétale témoin, dans laquelle une réponse immunitaire élevée de ladite plante ou cellule végétale indique que ledit composé candidat est un MAMP présent chez *Sclerotinia spp* ou *Botrytis ssp.*

8. Méthode d'identification par criblage selon la revendication 7, dans laquelle dans l'étape (iii) la réponse immunitaire de ladite plante ou cellule végétale est mesurée au moyen d'une évaluation de la production d'éthylène et/ou de l'expression de gènes répondeurs immunitaires ou de gènes rapporteurs.

9. Méthode de purification d'un MAMP présent chez *Sclerotinia spp* ou *Botrytis ssp,* comprenant l'utilisation d'une protéine AtRLP30, ou de parties extracellulaires de celle-ci, comprenant une séquence présentant une identité d'au moins 60 % avec SEQ ID No 1.

10. Méthode de sensibilisation d'une plante contre les infections fongiques, la méthode comprenant la mise en oeuvre chez ladite plante d'une méthode selon l'une quelconque des revendications 1 à 6.


### SEQUENCE LISTING

<110> Eberhard Karls Universitat Tübingen
<120> A Novel Immunogenic Fungal Extract and Pattern Recognition Receptor in Plants
<130> U30444PCT
<150> GB1219017.9
   <151> 2012 10 23
<160> 2
<170> PatentIn version 3.5
<210> 1
   <211> 786
   <212> PRT
   <213> Arabidopsis thaliana
<400> 1
   Met Ile Pro Ser Gln Ser Asn Ser Phe Ser Gly Ser Val Ile Thr Leu
   1               5                   10                  15
   Tyr Phe Phe Leu Leu Gly Ser Leu Val Leu Arg Thr Leu Ala Ser Ser
               20                  25                  30
   Arg Leu His Tyr Cys Arg His Asp Gln Arg Asp Ala Leu Leu Glu Phe
           35                  40                  45
   Lys His Glu Phe Pro Val Ser Glu Ser Lys Pro Ser Pro Ser Leu Ser
       50                  55                  60
   Ser Trp Asn Lys Thr Ser Asp Cys Cys Phe Trp Glu Gly Val Thr Cys
   65                  70                  75                  80
   Asp Asp Glu Ser Gly Glu Val Val Ser Leu Asp Leu Ser Tyr Val Leu
                   85                  90                  95
   Leu Asn Asn Ser Leu Lys Pro Thr Ser Gly Leu Phe Lys Leu Gln Gln
               100                 105                 110
   Leu Gln Asn Leu Thr Leu Ser Asp Cys His Leu Tyr Gly Glu Val Thr
           115                 120                 125
   Ser Ser Leu Gly Asn Leu Ser Arg Leu Thr His Leu Asp Leu Ser Ser
       130                 135                 140
   Asn Gln Leu Thr Gly Glu Val Leu Ala Ser Val Ser Lys Leu Asn Gln
   145                 150                 155                 160
   Leu Arg Asp Leu Leu Leu Ser Glu Asn Ser Phe Ser Gly Asn Ile Pro
                   165                 170                 175
   Thr Ser Phe Thr Asn Leu Thr Lys Leu Ser Ser Leu Asp Ile Ser Ser
               180                 185                 190
   Asn Gln Phe Thr Leu Glu Asn Phe Ser Phe Ile Leu Pro Asn Leu Thr
           195                 200                 205
   Ser Leu Ser Ser Leu Asn Val Ala Ser Asn His Phe Lys Ser Thr Leu
       210                 215                 220
   Pro Ser Asp Met Ser Gly Leu His Asn Leu Lys Tyr Phe Asp Val Arg
   225                 230                 235                 240
   Glu Asn Ser Phe Val Gly Thr Phe Pro Thr Ser Leu Phe Thr Ile Pro
                   245                 250                 255
   Ser Leu Gln Ile Val Tyr Leu Glu Gly Asn Gln Phe Met Gly Pro Ile
               260                 265                 270
   Lys Phe Gly Asn Ile Ser Ser Ser Ser Arg Leu Trp Asp Leu Asn Leu
           275                 280                 285
   Ala Asp Asn Lys Phe Asp Gly Pro Ile Pro Glu Tyr Ile Ser Glu Ile
       290                 295                 300
   His Ser Leu Ile Val Leu Asp Leu Ser His Asn Asn Leu Val Gly Pro
   305                 310                 315                 320
   Ile Pro Thr Ser Ile Ser Lys Leu Val Asn Leu Gln His Leu Ser Leu
                   325                 330                 335
   Ser Asn Asn Thr Leu Glu Gly Glu Val Pro Gly Cys Leu Trp Gly Leu
               340                 345                 350
   Met Thr Val Thr Leu Ser His Asn Ser Phe Asn Ser Phe Gly Lys Ser
           355                 360                 365
   Ser Ser Gly Ala Leu Asp Gly Glu Ser Met Gln Glu Leu Asp Leu Gly
       370                 375                 380
   Ser Asn Ser Leu Gly Gly Pro Phe Pro His Trp Ile Cys Lys Gln Arg
   385                 390                 395                 400
   Phe Leu Lys Tyr Leu Asp Leu Ser Asn Asn Leu Phe Asn Gly Ser Ile
                   405                 410                 415
   Pro Pro Cys Leu Lys Asn Ser Thr Tyr Trp Leu Lys Gly Leu Val Leu
               420                 425                 430
   Arg Asn Asn Ser Phe Ser Gly Phe Leu Pro Asp Val Phe Val Asn Ala
           435                 440                 445
   Ser Met Leu Leu Ser Leu Asp Val Ser Tyr Asn Arg Leu Glu Gly Lys
       450                 455                 460
   Leu Pro Lys Ser Leu Ile Asn Cys Thr Gly Met Glu Leu Leu Asn Val
   465                 470                 475                 480
   Gly Ser Asn Ile Ile Lys Asp Thr Phe Pro Ser Trp Leu Val Ser Leu
                   485                 490                 495
   Pro Ser Leu Arg Val Leu Ile Leu Arg Ser Asn Ala Phe Tyr Gly Ser
               500                 505                 510
   Leu Tyr Tyr Asp His Ile Ser Phe Gly Phe Gln His Leu Arg Leu Ile
           515                 520                 525
   Asp Ile Ser Gln Asn Gly Phe Ser Gly Thr Leu Ser Pro Leu Tyr Phe
       530                 535                 540
   Ser Asn Trp Arg Glu Met Val Thr Ser Val Leu Glu Glu Asn Gly Ser
   545                 550                 555                 560
   Asn Ile Gly Thr Glu Asp Trp Tyr Met Gly Glu Lys Gly Pro Glu Phe
                   565                 570                 575
   Ser His Ser Asn Ser Met Thr Met Ile Tyr Lys Gly Val Glu Thr Asp
               580                 585                 590
   Phe Leu Arg Ile Pro Tyr Phe Phe Arg Ala Ile Asp Phe Ser Gly Asn
           595                 600                 605
   Arg Phe Phe Gly Asn Ile Pro Glu Ser Val Gly Leu Leu Lys Glu Leu
       610                 615                 620
   Arg Leu Leu Asn Leu Ser Gly Asn Ser Phe Thr Ser Asn Ile Pro Gln
   625                 630                 635                 640
   Ser Leu Ala Asn Leu Thr Asn Leu Glu Thr Leu Asp Leu Ser Arg Asn
                   645                 650                 655
   Gln Leu Ser Gly His Ile Pro Arg Asp Leu Gly Ser Leu Ser Phe Leu
               660                 665                 670
   Ser Thr Met Asn Phe Ser His Asn Leu Leu Glu Gly Pro Val Pro Leu
           675                 680                 685
   Gly Thr Gln Phe Gln Ser Gln His Cys Ser Thr Phe Met Asp Asn Leu
       690                 695                 700
   Arg Leu Tyr Gly Leu Glu Lys Ile Cys Gly Lys Ala His Ala Pro Ser
   705                 710                 715                 720
   Ser Thr Pro Leu Glu Ser Glu Glu Phe Ser Glu Pro Glu Glu Gln Val
                   725                 730                 735
   Ile Asn Trp Ile Ala Ala Ala Ile Ala Tyr Gly Pro Gly Val Phe Cys
               740                 745                 750
   Gly Leu Val Ile Gly His Ile Phe Phe Thr Ala His Lys His Glu Trp
           755                 760                 765
   Phe Met Glu Lys Phe His Arg Asn Lys Arg Arg Val Val Thr Thr Ser
       770                 775                 780
   Ala Arg
   785
<210> 2
   <211> 2553
   <212> DNA
   <213> Arabidopsis thaliana
<400> 2
   ataacctgtt acttacaaaa caaacaaaat gattccaagc caatctaatt ccttttctgg      60
   tagtgttatt accttgtatt tcttcctcct gggttccctt gttctacgca ctcttgcttc     120
   ttctaggctt cactattgcc gccatgacca aagggatgct cttctcgagt tcaaacacga     180
   gtttccggta agtgaatcga agccaagtcc atcgttgagt tcatggaaca agactagtga     240
   ttgctgtttt tgggagggtg tcacgtgcga tgatgaatct ggcgaggtgg tttcacttga     300
   ccttagttat gtccttctca acaactcttt gaaaccaact agtggtcttt tcaaactcca     360
   acaactccag aacctgactc tcagtgattg ccatctctat ggagaggtta cttcttcact     420
   aggaaacctt tctcgtctca cgcatcttga cctttcgagt aatcagctga caggtgaagt     480
   tctggcttcg gtcagtaagc taaaccaact tagagacctt ttactttccg aaaacagttt     540
   tagtggtaac attcctactt catttaccaa tttaacgaag ctttctagtt tagacatctc     600
   tagcaatcag ttcacattgg aaaatttctc tttcatacta ccaaatttaa ccagcttgtc     660
   ctccttaaac gttgcctcta atcactttaa atccacgctt ccatctgata tgagtggact     720
   ccacaacttg aaatattttg atgtgcgtga gaattcattt gtcgggactt ttcctacatc     780
   cttattcacg attccttcgt tacaaattgt ttatttggaa ggaaaccagt tcatgggacc     840
   tataaagttt gggaatatat cttcatcttc taggctttgg gatctaaacc ttgctgataa     900
   caaattcgat gggccaatcc ctgaatatat atctgaaatt cacagtctca tagtactaga     960
   tcttagccac aacaacttag ttgggccaat ccccacttct atatcaaagt tggtcaacct    1020
   tcagcatctt agtctttcaa acaatacctt ggaaggcgaa gtgccaggtt gcttatgggg    1080
   attgatgaca gtgacacttt cccacaattc tttcaacagt tttggaaagt catcatccgg    1140
   agctttggat ggagaatcaa tgcaggagtt ggatcttggt tcgaattcac ttggaggacc    1200
   ttttccccat tggatctgca agcaaaggtt cttaaagtac ttagacttgt ccaacaatct    1260
   cttcaacggc tcaattcctc cttgtttgaa aaattccact tattggctta aagggctagt    1320
   tctgcgtaac aacagcttca gcggatttct cccagacgta tttgtcaatg ctagcatgtt    1380
   attatcactt gacgttagct acaaccggtt ggagggaaaa cttccaaaat ccctgatcaa    1440
   ttgcactggt atggaacttc tgaatgtggg aagcaacata atcaaggaca cttttccatc    1500
   ctggttggtt tctttgccat cattacgtgt cctcatcctc agatctaatg cattctatgg    1560
   gtcgttatat tatgaccaca tatcttttgg gtttcaacat ttgagactca ttgatatatc    1620
   acagaatggc ttcagtggaa ctttgtcacc tttatatttc tccaattggc gtgagatggt    1680
   gacatctgtc ttagaagaaa acggctctaa tataggtaca gaggattggt acatgggcga    1740
   gaaagggccc gagttcagcc atagtaattc gatgactatg atatataaag gagtagaaac    1800
   ggacttcttg cggatcccat atttcttcag agcattgact tttctggaaa cagatttttt    1860
   gggaatatac ctgaatccgt tggtttgttg aaggaattgc gtcttctcaa tttgtcaggt    1920
   aactcattca caagcaatat ccctcagtca ttggcaaatt tgacaaatct tgagacattg    1980
   gacctatccc ggaatcagct atcaggtcac atccctcgag atcttggtag cctctctttt    2040
   ctgtcgacca tgaatttctc ccataacctt ctcgaaggtc cagttccact aggcactcag    2100
   tttcaaagcc aacattgttc tacattcatg gacaacctca gactctacgg tcttgagaaa    2160
   atctgtggaa aagctcatgc cccgagttct acaccactag aatccgagga attttcggaa    2220
   ccagaagaac aagtgattaa ctggatagca gcggcaatag cctacggacc tggtgtgttt    2280
   tgtggattag ttattggaca tatcttcttt actgcacaca aacacgagtg gttcatggaa    2340
   aaattccatc gaaacaagcg cagagtagtc accacaagtg ctcgttgaac accatcacct    2400
   ctcacaattt ctctgagtga ttgtgaaacg tatatgtttg ttatgatatc tgtcagttgt    2460
   gttatgcata aactctttgt ctttgtaatt ttctacttta tggtattgta agaaacggct    2520
   ttggtggtat ctgatcttct atatgttggg ttg                                 2553